# EUROPEAN PATENT APPLICATION

(11) **EP 1 050 306 A1**
(43) Date of publication of application: **08.11.2000**
(21) Application number: 99901204.0
(22) Date of filing: 01.02.1999
(51) Int. Cl.: A61K 38/02

(54) **CYTOKINE INDUCERS COMPRISING M161Ag**

(30) Priority: 30.01.1998 JP 3238498
(71) Applicant: Japan Science and Technology Corporation, Kawaguchi-shi, Saitama 332-0012 (JP)
(72) Inventor: SEYA, Tsukasa, Nara-shi, Nara 631-0022 (JP); MATSUMOTO, Misako, Ikoma-shi, Nara 630-0121 (JP)
(74) Representative: Cresswell, Thomas Anthony
(86) International application number: JP9900414
(87) International publication number: WO9938523

(57) **Abstract**

Cytokine inducers comprising a membrane protein M161Ag which is contained in cells latently infected with *Mycoplasma fermentans* such as a human myelocytic leukemia cell line P39(+). These cytokine inducers comprising the membrane protein M161Ag or gene recombination products thereof are useful as immunomodulators or remedies for various immunological diseases.

## Description

### Technical Field

This invention relates to cytokine inducers comprising protein M161Ag, immunomodulators and immunotherapeutic agents.

### Background Arts

M161Ag is a membrane protein which is contained in cells latently infected with *Mycoplasma fermentans* such as a human myelocytic leukemia cell line P39(+), and has functions such as activation of the alternative pathway and adsorption of the complement C3. Isolation and purification of this protein and preparation of monoclonal antibody have already been retorted [Matsumoto et al., J. Exp. Med. 181, 115-125 (1995)]. Further, the primary structure has almost been reported [Nature Med., 3: 1266-1270 (1997)](Japanese Patent Unexamined Publication No. Hei 9-157295).

*Mycoplasma fermentans (M. fermentans)* is an intracellular parasitic bacterium, which appears to positive under the disease states of immunosuppressive conditions, for example, HIV infected patients, patients with cancer such as leukemia and myeloma and patients with aplastic anemia. Main parasitic host is identified in vitro as human tumor cell strain. It is known that in a positive case of *M. fermentans*, M161Ag is essentially positive.

In the report by the another group, Mycoplasma is a cause of lymphopenia and is a cofactor of AIDS crisis. Further, *M. fermentans* has been suggested to induce cytokines as a result of stimulation of leukocytes system in vitro. These phenomena are not generated by an action of other Mycoplasma species and are observed in *M. fermentans.* Consequently, a gene product specific to *M. fermentans* might instruct lymphopenia and cytokine induction. However, the fact that what substance may be involved in those actions is unknown. We have identified a substance, which was involved in these biological activities of *M. fermentans*, by an assay using purified authentic sample, and demonstrated the range of the biological activities, thereby completed the present invention.

An object of the present invention is to provide cytokine inducers, immunomodulators and immunotherapeutic agents comprising M161Ag by applying physiological activity and cytokine inducing activity of M161Ag, and to make good use of treatment for various immunological diseases.

### Disclosure of Invention

We have succeeded, using purified authentic sample of M161Ag, to induce effectively the inflammatory cytokines such as IL-1β, TNF-α and IL-6 and the lymphocyte-activating cytokines such as IL-10 and IL-12 by stimulating immune competent cells such as monocytes and lymphocytes, and to remove infected cells as a result of apoptosis, and completed the present invention.

The present invention relates to cytokine inducers, immunomodulators or remedies for various immunological diseases comprising the protein M161Ag or gene recombination products thereof.

In addition, the present invention relates to therapeutic method for diseases caused by cytokine deficiency, immunological diseases and immune diseases comprising administering therapeutically sufficient amounts of the protein M161Ag or gene recombination products thereof. Further, the present invention relates to use of the protein M161Ag or gene recombination products thereof for production of cytokine inducers, immunomodulators or remedies for immunological diseases.

DNA of M161Ag of the present invention can be obtained as genomic DNA from human myelocytic leukemia cell line P39 and human fibroblast cell line W138, in which *M. fermentans* is infected and proliferated. Since these DNAs contain five codons of TGA (termination codon in E. coli and human; and tryptophan in Mycoplasma), these codons are replaced by TGG, and the resultant sequence is inserted into the expression vector of E. coli having His-tag such as pET, then is forcibly expressed in cells. The thus obtained large amount of gene recombination products is purified by nickel Sepharose column. The obtained sample is confirmed as E. coli derived pyrogen-free.

Thus purified M161Ag has 45kDa of molecular weight and is bound with lipid such as palmitic acid in N-terminal cysteine. When M161Ag is mixed with cells, a part of M161Ag is incorporated into cells by an action of this lipid. When M161Ag is mixed with peripheral blood, monocytes and monocyte system cells in vitro, the inflammatory cytokines derived from macrophage such as IL-1β, TNF-α and IL-6, and cytokines having modulator activity of lymphocytes such as IL-10 and IL-12 can be identified in the cultured supernatant solution within 24 hours. From comparison of an activity of M161Ag with that of lipopolysaccharide (LPS), in case of using identical weight (such as 10 ng) of these substances, all of the above cytokines are found to be more potentially induced by M161Ag than by LPS. Monocytes used in this experiment are prepared by the method of Karp et al. using elutriation system (Beckman) and have purification above 95%. Consequently, it can be concluded that these cytokines were induced by direct stimulation of M161Ag to monocytes.

It can be proved that M161Ag is a bioactive substance, which potentially induces human cytokines, derived from Mycoplasma. In addition, it has been elucidated that M161Ag activated human fundamental immune system (innate immunity) through this action and improved host immunity in the immunosuppressive condition, further resulted the infected cells such as lymphocytes to apoptotic death, and suppressed disease state caused by excess immune activation such as allergy.

As explained hereinabove, it is proved that M161Ag induces cytokines as a modulator in various immune system through the monocyte system.

### Brief Description of Drawing

Fig. 1 indicates inducing action of the protein M161Ag of the present invention on IL-1β, TNF-α and IL-6 in the monocyte cell THP-I. In Fig. 1, the left columns show data from cell lysates and the right columns show data in the conditioned medium.
Fig. 2 indicates inducing action of the protein M161Ag of the present invention on IL-1β, TNF-α and IL-6 in the purified blood monocytes. In Fig. 2, the left columns show data from cell lysates and the right columns show data in the conditioned medium.
Fig. 3 indicates inducing action of the protein M161Ag of the present invention on IL-10 (Fig. 3, left) and IL-12 (Fig. 3, right).

### Best Mode of Carrying Out the Invention

The cytokines induced by the protein M161Ag of the present invention are known to have various bioactivities. For example, IL-1 is known to have activities of T cell activation, neutrophil activation, stimulation of antitumor activity, proliferation of fibroblasts and increase of ACTH and GH. TNF-α is a factor for involving in proliferation and differentiation of cells, and especially is a factor having tumor necrotizing action. It is reported also to increase production of prostaglandins and platelet activating factor, further to have antiviral action.

IL-6 has actions to stimulate differentiation of B cells to antibody production cells as well as to stimulate differentiation and proliferation of T cell and macrophage. IL-10 is a factor having an action to proliferate T cell. IL-12 is a factor having actions to activate cytotoxic T cell (CTL) and NK cell.

M161Ag can be applied its cytokine inducing activity for treatment of various immunological diseases and cancers.

Since the active component of the present invention is to activate human innate immunity system, it is useful for remedies for various immunological diseases involved in the innate immunity system. Examples of immunological diseases are, for example, allergic diseases and autoimmune diseases.

The protein M161Ag of the present invention can be the extract of cells such as cell line P39(+), and is also able to be the expression product (gene recombination product) by using DNA with various host cells as disclosed in Japanese Patent Unexamined Publication No. Hei 9-157295.

In a sequence of the protein M161Ag of the present invention, one or more amino acids may be deleted; one or more amino acids may be substituted to the other amino acids; or one or more amino acids may be added, within maintaining its activity.

The M161Ag of the present invention can be used in the form of protein itself, in addition to that, it can be used in the form that the protein is modified with fatty acid or other substances in the N-terminal or the suitable position in the amino acid sequence, or the protein is modified by binding with specific antibody to cells such as cancer cells.

Amount of administration of the protein M161Ag of the present invention as an active ingredient depends on conditions of patients and types of diseases, and is usually 0.01 - 100 ng/kg/day, preferably 0.1 - 10 ng/kg/day for 1 - 3 times/day.

Routes of administration are preferably intravenous injection and subcutaneous injection, however there is no specific limitation if administered parenterally such as using sublingual tablets and suppositories.

Preparations prepared by conventional protein formulations can be used in the administration form. Emulsion such as liposome preparation can also be used.

### Examples

The present invention is explained more concretely by the following examples, but is not construed as limiting by these examples.

### Example 1

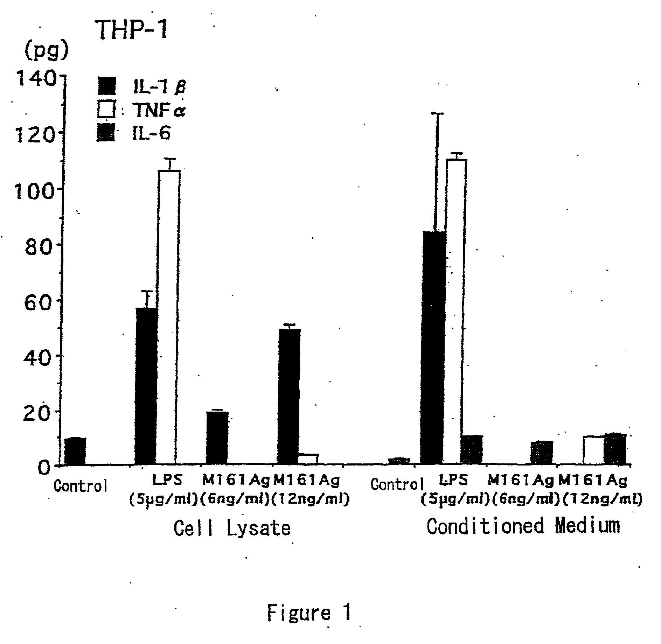

M161Ag, 6 ng and 12 ng, respectively, was added to the monocyte cell system line THP-1 (10⁶). Culture supernatant was collected after 24 hours and various cytokines were quantitatively assayed by sandwich ELISA. Fig. 1 shows results of assay as well as comparative data of using LPS.

### Example 2

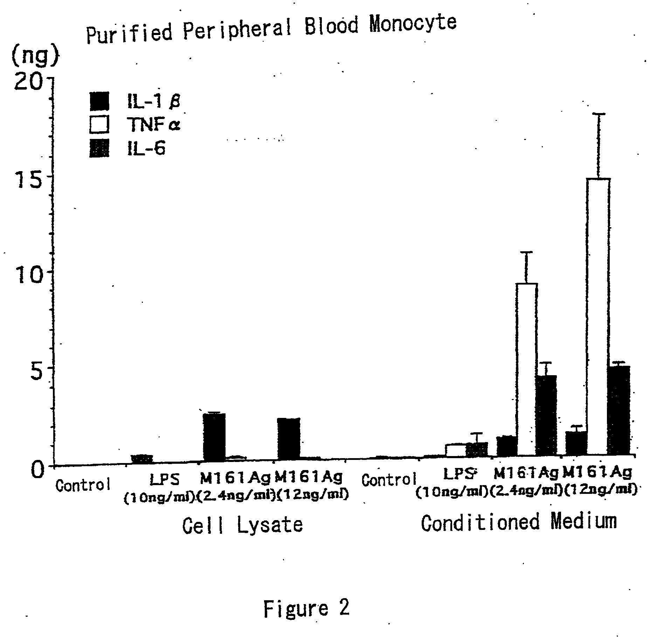

M161Ag, 2.4 ng and 12 ng, respectively, was added to the purified peripheral blood monocytes (10⁶). Culture supernatant was collected after 24 hours and various cytokines were quantitatively assayed by sandwich ELISA. Fig. 2 shows results of assay as well as comparative data of using LPS.

### Example 3

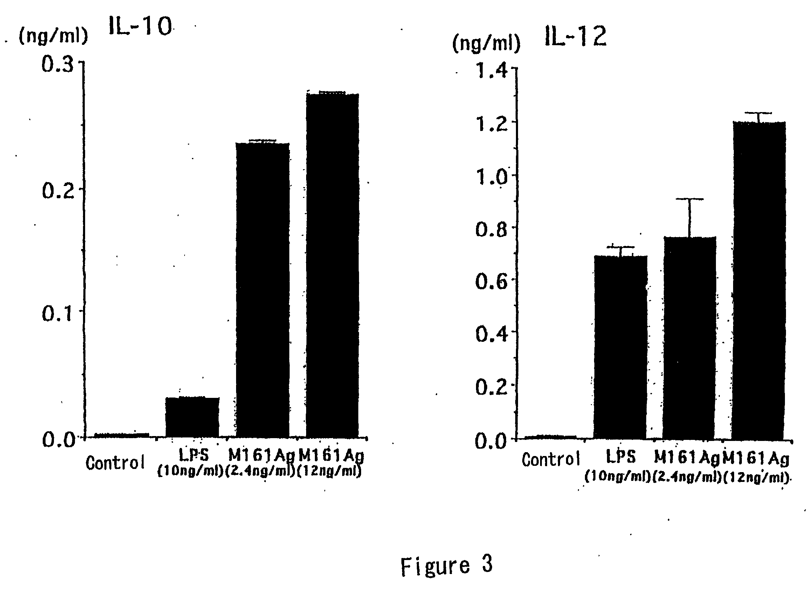

M161Ag, 2.4 ng and 12 ng, respectively, was added to the purified peripheral blood monocytes (10⁶). After 24 hours, Il-10 and IL-12 were quantitatively assayed. Results are shown in Fig. 3.

### Industrial Applicability

The present invention provides pharmaceutical agent comprising novel bioactivity of membrane protein M161Ag of *Mycoplasma fermentans.* M161Ag shows potential cytokine inducing activities and can be applied as immunomodulators for allergic and immunosuppressive conditions.

## Claims

1. Cytokine inducers comprising a protein M161Ag or gene recombination products thereof.

2. The cytokine inducers according to claims 1 wherein induced cytokines are IL-1β, TNF-α, IL-6, IL-10, IL-12 and/or INF-γ.

3. The cytokine inducers according to claim 1 or 2 wherein the cytokine inducers are used as immunomodulators.

4. The cytokine inducers according to claim 3 wherein the immunomodulators are used as remedies for immunological diseases.

5. The cytokine inducers according to any one of claims 1 - 4 wherein the protein M161Ag or gene recombination products thereof are acylated with fatty acid in N-terminal thereof.

6. A remedy for immunological diseases comprising protein M161Ag or gene recombination products thereof.

7. A therapeutic method for diseases caused by cytokine deficiency comprising administering therapeutically sufficient amount of protein M161Ag or gene recombination products thereof.

8. A therapeutic method for immunological diseases comprising administering therapeutically sufficient amount of protein M161Ag or gene recombination products thereof.

9. Use of protein M161Ag or gene recombination products thereof for production of cytokine inducers.

10. Use of protein M161Ag or gene recombination products thereof for production of remedies for immunological diseases.
